# EUROPÄISCHE PATENTSCHRIFT

(11) **EP 4 052 671 B1**
(45) Veröffentlichungstag und Bekanntmachung des Hinweises auf die Patenterteilung: **07.08.2024**
(21) Anmeldenummer: 22159128.2
(22) Anmeldetag: 28.02.2022
(51) Int. Cl.: A61B 17/50, A61B 17/30, A61B 17/28

(54) **PINZETTENARTIGE VORRICHTUNG**
TWEEZER-STYLE DEVICE
DISPOSITIF DE TYPE PINCETTE

(30) Priorität: 02.03.2021 DE 102021104959
(43) Veröffentlichungstag der Anmeldung: 07.09.2022
(73) Patentinhaber: Pfeiffer, Josef, 82494 Krün (DE)
(72) Erfinder: PFEIFFER, Josef, 82494 Krün (DE); SPAVOR, Jakub, 82494 Krün (DE); KRIVANIC, Dusan, 82494 Krün (DE)
(74) Vertreter: Flosdorff, Jürgen

(56) Entgegenhaltungen:
- DE-A1- 102018 007 593
- DE-A1- 19 860 172
- US-A- 6 106 041
- US-A1- 2016 000 423

## Beschreibung

Die Erfindung betrifft eine pinzettenartige Vorrichtung zum Entfernen von Zecken aus der Haut, mit einer äußeren Hülse, in der eine Pinzette verschieblich gehalten ist, die zwei elastisch auseinander gespreizte Pinzettenhälften aufweist, die einstückig mit einem rückwärtigen Griffabschnitt ausgebildet sind, dessen Endabschnitt aus der Hülse herausragt, wobei die Pinzettenhälften aufeinander zulaufende Endabschnitte zum Ergreifen einer Zecke haben, und wobei die Endabschnitte in einem Ausgangszustand der Pinzette mit Abstand voneinander vorne aus der Hülse heraus ragen, wobei die Endabschnitte beim Andrücken der Hülse an die Haut durch den vorderen Rand der Hülse zusammengezwängt werden, um die Zecke ergreifen zu können.

Derartige Vorrichtungen werden auch als Zeckenzangen bezeichnen und ermöglichen es, mit ihren abgewinkelten Endabschnitten eine Zecke zu ergreifen, die daraufhin aus der Haut herausgezogen werden kann. Danach gibt eine herkömmliche Zeckenzange die Zecke wieder frei, indem die Zeckenzange aufgrund der Federkraft der auseinandergepreizten Pinzettenhälften aus der äußeren Hülse herausgleitet, so dass die Zecke entsorgt werden kann.

Je nach Infektion einer Zecke können verschiedene Erkrankungen auf den Menschen (und auf ein Tier) übertragen werden. Die häufigste Erkrankung ist die Borreliose, wobei bis zu 30% aller Zecken mit Borrelien infiziert sind. Eine andere durch Zecken übertragene Krankheit ist die FSME, die ebenfalls zu schweren gesundheitlichen Schäden führen kann. Zecken können auch andere Krankheiten wie Ehrlichiose übertragen.

Solche und weitere von Zecken übertragene Krankheiten breiten sich erschreckend schnell aus und müssen nach einem Zeckenstich möglichst schnell zielgenau behandelt werden, um die Krankheitserreger abtöten zu können, bevor die Krankheit richtig ausbricht. Deshalb wäre es wichtig, nach einem Zeckenstich festzustellen, ob die Zecke infiziert war und welche Krankheitserreger sie abgegeben hat. Dies kann in einem entsprechend ausgerüsteten Labor ermittelt werden, wenn das Labor die Zecke zur Untersuchung erhält.

Aus der DE 10 2018 007 593 A1 ist eine Vorrichtung der eingangs genannten Art bekannt, mit der nicht nur Zecken durch eine Drehbewegung oder durch Abhebeln aus der Haut entfernt werden können, sondern die Zecken werden in der Zeckenzange fixiert und in der äußeren Hülse, die durch einen Deckel verschließbar ist, eingeschlossen, wobei die äußere Hülse eine Verpackung für die Zecke bildet, so dass diese auf bequeme Weise an ein Labor versendbar ist, bei der die Erreger der Zecke ermittelt werden können.

Bei dieser bekannten Zeckenzange ist in der Innenwand der Hülse eine Vertiefung ausgebildet, in die ein an der Pinzette ausgebildeter seitlicher Vorsprung einrasten kann, wobei Vertiefung und Vorsprung auch auf umgekehrte Weise ausgebildet sein können. Dabei kann es passieren, dass sich diese Arretierung z.B. beim Transport der pinzettenartigen Vorrichtung löst und die Zecke freigegeben wird, so dass sie nicht zur Untersuchung in ein Labor gelangt. Es ist auch möglich, dass sich die Zecke aus den Pinzettenhälften befreit und aus der rückwärtigen Öffnung der Hülse entkommt.

Die US 2016/000423 A1 befasst sich mit der Halterung einer Nähnadel, mit der beispielsweise bei einem operativen Eingriff die damit einhergehende Wunde vernäht wird. Die Nähnadel-Halterung enthält eine Pinzetten-artige Klemmvorrichtung für die Nadel, die in den beiden spitz zulaufenden Klemmgliedern Einbuchtungen hat. Die Klemmvorrichtung ist feststehend in einer äußeren Hülse angeordnet, in der eine Innenhülse sitzt, die mittels eines Betätigungsglieds verschieblich in der äußeren Hülse angeordnet ist, wozu das Betätigungsglied in einem Längsschlitz der äußeren Hülse sitzt. In der nach vorne ausgefahrenen Position der Innenhülse drückt diese die beiden Klemmglieder zusammen, während sich in der zurückgezogenen Position der Innenhülse die Klemmvorrichtung öffnet, so dass eine Nähnadel eingelegt werden kann. Aus der Innenhülse ist ein federnder Steg ausgeschnitten, der an seinem Ende einen dachförmigen Vorsprung aufweist, der in eine ebenfalls dachförmige Verzahnung an der äußeren Hülse eingreifen kann. Beim Verschieben der Innenhülse gleitet der dachförmige Vorsprung über die Verzahnung hinweg. Durch Eingriff in die Verzahnung an einer ausgewählten Stelle kann die Klemmkraft der beiden Klemmglieder eingestellt werden.

Die DE 198 60 172 A1 offenbart eine automatische Zeckenzange, die mit einem Drehantrieb und einem zusätzlichen Linearantrieb versehen ist, mit dem die Zeckenzange linear von der Haut weg bewegbar ist.

Die Zeckenzange der US 6,106,041 hat einen inneren Mechanismus mit geneigten Flächen, die beim Andrücken der Pinzette an die Haut eine elastische Klemmkraft hervorrufen.

Der vorliegenden Erfindung liegt die Aufgabe zugrunde, eine pinzettenartige Vorrichtung der betrachteten Art so auszubilden, dass sie es einem Benutzer nicht nur auf einfache und bequeme Weise ermöglicht, eine Zecke zu ergreifen, sondern auch sicherzustellen, dass die ergriffene Zecke nicht aus der äußeren Hülse entkommen kann.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, dass die in der Hülse in die Endlage zurückgezogene Pinzette durch eine Rasteinrichtung verrastet ist, die nur durch Einwärtsdrücken einer aus der Wand der Hülse freigeschnittene, federnde Zunge freizugeben ist.

In dem spannungslosen Ausgangszustand der Pinzette ragen die aufeinander zulaufenden Endabschnitte der Pinzettenhälften vorne aus der äußeren Hülse heraus. Die Pinzettenhälften werden zum Ergreifen einer Zecke seitlich zusammengedrückt, indem die Pinzette auf die Haut aufgesetzt und durch Druck in die Hülse zurück bewegt wird, wobei die auseinandergespreizten vorderen Endabschnitte auf den Rand der äußeren Hülse auflaufen und von diesem Rand aufeinander zu gedrückt werden, bis sie die Zecke fest ergreifen. Danach wird die Pinzette in der Hülse durch die erfindungsgemäße Rasteinrichtung arretiert, die nur durch Einwärtsdrücken einer aus der Wand der Hülse freigeschnittenen federnden Zunge frei gegeben werden kann. Diese Arretierung in der Hülse kann sich nicht selbsttätig oder unabsichtlich durch äußere Einwirkung lösen, da hierzu ein seitlicher Druck auf einen bestimmten, kleinen Bereich der Umfangswand der äußeren Hülse ausgeübt werden muss, was in der Praxis nur mit Absicht, nicht aber versehentlich erfolgen kann.

Weiter sieht die Erfindung vor, dass die Rasteinrichtung einen von dem Griffabschnitt der Pinzette freigeschnittenen, federnden Steg aufweist, der an seinem freien Ende eine Rastnase hat, die im entspannten Zustand des Stegs über die Außenkontur des Griffabschnitts hinaus ragt und radial nach innen einen Freiraum mindestens in der Breite der Rastnase hat, so dass die Rastnase in die Außenkontur des Griffabschnitts hinein gedrückt werden kann. Dabei hat die Rastnase eine etwa rechtwinklige vordere Stirnfläche, die sicher in einer Aussparung gehalten ist, die in der Wand der Hülse in axialer Richtung vor deren freigeschnittenen Zunge ausgebildet ist. Auch wenn auf den stabförmigen Griffabschnitt eine nach vorne gerichtete Kraft ausgeübt würde, könnte hierdurch die Rastnase nicht aus der Aussparung austreten.

Dies ist nur durch einwärts gerichteten Druck auf die federnde Zunge der Hülse möglich, die den freigeschnittenen Steg bei der in die Hülse zurückgezogenen Position der Pinzette im wesentlichen bis zu der Rastnase hin überdeckt.

In weiterer Ausgestaltung der Erfindung ist vorgesehen, dass der Griffabschnitt querschnittlich in der Hülse nicht-drehbar geführt ist. Hierzu hat der Griffabschnitt vorzugsweise eine Rechteckform, während die Hülse einen rückwärtigen Abschnitt mit einem entsprechenden rechteckigen Hohlraum aufweist. Andere Querschnittsformen, die eine Nichtdrehbarkeit zur Folge haben, liegen im Rahmen der Erfindung.

Zweckmäßigerweise ist auf den Endabschnitt des Griffabschnitts eine radial überstehende Kappe aufgesetzt, die beim Vorschub der Pinzette zum Austritt der vorderen Abschnitte der Pinzettenhälften aus der Hülse an deren rückwärtigen Randkante anliegt, wodurch die vorgeschobene Endposition der Pinzette begrenzt ist. In der zurückgezogenen Position befinden sich die Pinzettenhälften vollständig innerhalb der Hülse.

Die Hülse hat nach einem weiteren Vorschlag den oben erwähnten rückwärtigen Abschnitt mit rechteckigem Hohlraum und einen anschließenden vorderen Abschnitt, der vorzugsweise querschnittlich einen kreisrunden Hohlraum hat. In diesem Zusammenhang wird mit großem Vorteil vorgeschlagen, dass an dem Griffabschnitt der Pinzette vor dem freigeschnittenen Steg ein ringförmiger Flansch angeformt ist, dessen Rand einen so kleinen Abstand von der Innenwand des vorderen Abschnitts der Hülse hat, dass die Pinzette zwar noch frei axial bewegbar in der Hülse ist, jedoch der hintere Innenraum der Hülse gegen den Durchgang einer Zecke abgedichtet ist, die sich aus den vorderen Endabschnitten der Zeckenhälften befreien konnte. Damit ist die Zecke vollkommen sicher in der Hülse gefangen und gelangt zuverlässig ins Labor zur Untersuchung auf Krankheitserreger.

Weitere Einzelheiten der Erfindung ergeben sich aus der nachfolgenden Beschreibung einer bevorzugten Ausführungsform der erfindungsgemäßen pinzettenartigen Vorrichtung. Dabei zeigen:

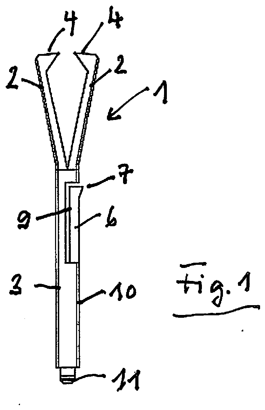

- Figur 1: eine Seitenansicht einer Pinzette der pinzettenartigen Vorrichtung;
- Figur 2: die Pinzette der Figur 1 innerhalb einer zugehörigen Hülse in einer Aufsicht und in einem Längsschnitt A-A;
- Figur 3: die Anordnung gemäß Figur 2, wobei die Pinzette in die rückwärtige Endlage zurückgezogen ist;
- Figur 3A: eine vergrößerte Darstellung mit der zurückgezogenen Pinzette;
- Figur 4: eine vergrößerte Darstellung des Bereichs B in Figur 3;
- Figuren 5A und 5B: eine abgewandelte Ausführungsform.

Die Pinzette 1 besteht aus zwei elastisch auseinander gespreizten Pinzettenhälften 2 und einem stabförmigen Griffabschnitt 3, wobei die Pinzette einstückig im Spritzgussverfahren aus Kunststoff hergestellt ist. Die Pinzettenhälften 2 haben aufeinander zu laufende Endabschnitte 4, die beim Zurückziehen der Pinzette 1 in der zugehörigen Hülse 5 zusammen gezwängt werden.

Aus dem stabförmigen Griffabschnitt 3 ist ein in Längsrichtung des Griffabschnitts verlaufender Steg 6 freigeschnitten, der an seinem freien Ende eine nach außen ragende Rastnase 7 hat, deren vordere Stirnfläche 8 rechtwinklig zur Längsachse des stabförmigen Griffabschnitts 3 verläuft. Die zwischen dem Steg 6 und dem verbleibenden Querschnitt des stabförmigen Griffabschnitts 3 ausgebildete Nut 9 ist so breit, dass die über die Außenkontur 10 vorstehende Rastnase 7 soweit zurück gedrückt werden kann, dass sie nicht mehr über die Außenkontur 10 vorsteht.

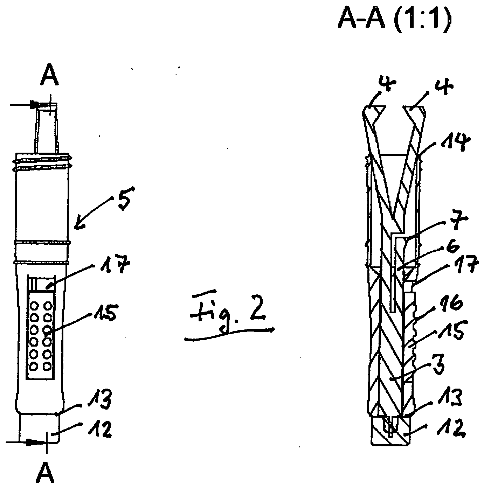

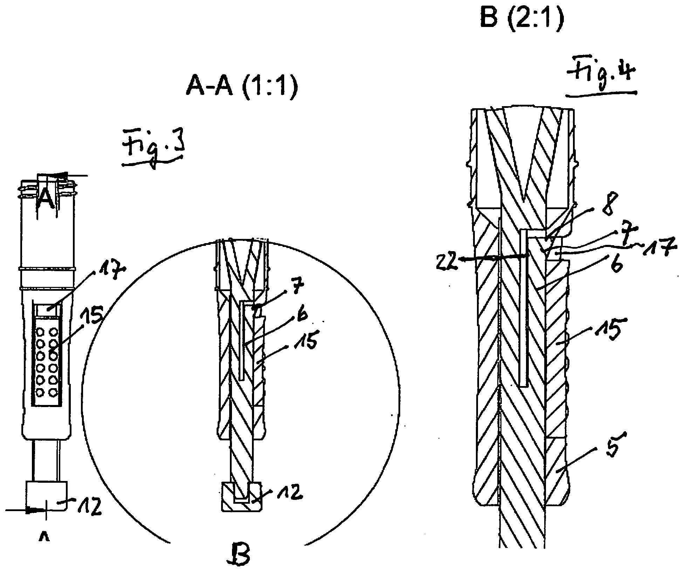

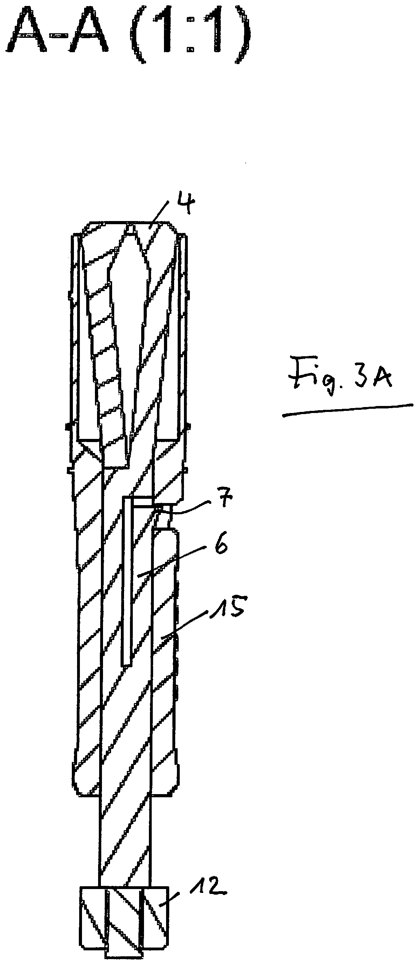

Figur 2 zeigt in einer Aufsicht und einem Längsschnitt den zusammengesetzten Zustand der Pinzette 1 mit der Hülse 5. Die Pinzette 1 ist an ihrem unteren Ende 11 mit einer Kappe 12 versehen, die in dem vorgeschobenen Zustand der Pinzette 1 an der rückwärtigen Randkante 13 der Hülse 5 anliegt. In dieser Lage ragen die Pinzettenhälften 2 aus dem vorderen Rand 14 hinaus, wobei sie so auseinander gespreizt sind, dass sie an dem Rand 14 anliegen können. Ihre nach innen weisenden Endabschnitte 4 sind voneinander beabstandet. Aus der Wand der Hülse 5 ist eine federnde Zunge 15 freigeschnitten, die zur besseren Handhabung auf ihrer Außenseite mit noppenartigen Vorsprüngen 16 versehen ist. Vor dem freien Ende der elastisch federnden Zunge 15 befindet sich eine Aussparung 17, in die die Rastnase 7 des Griffabschnitts 3 der Pinzette 1 einrastet, wenn die Pinzette 1 in die in den Figuren 3 und 4 dargestellte Lage zurückgezogen wird.

Wie diese Figuren zeigen, überdeckt die federnde Zunge 15 den freigeschnittenen Steg 6 bis zu der abgewinkelten Rastnase 7, die in die Aussparung 17 eingerastet ist. In dieser rückwärtigen Position der Pinzette 1 in der Hülse 5 sind die aufeinander zu gezwängten Endabschnitte 4 vollständig in die Hülse 5 zurück gezogen, wobei ihre Spitzen im wesentlichen aneinander anliegen, so dass sie zwischen den Endabschnitten 4 eine Zecke einklemmen können.

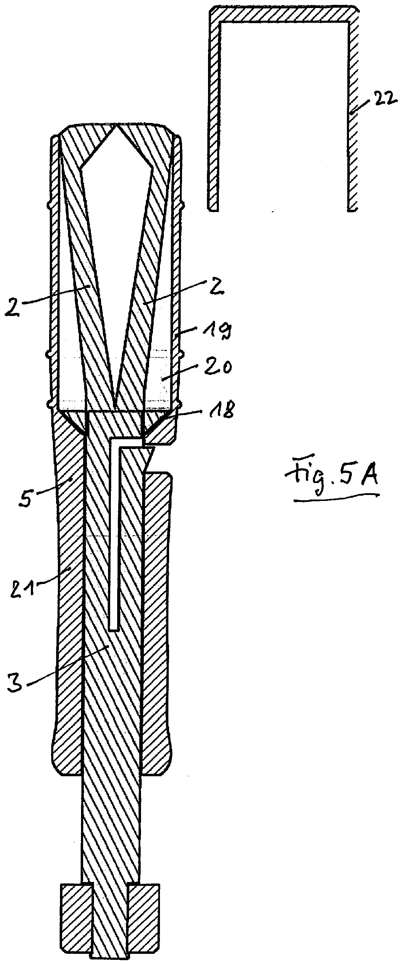

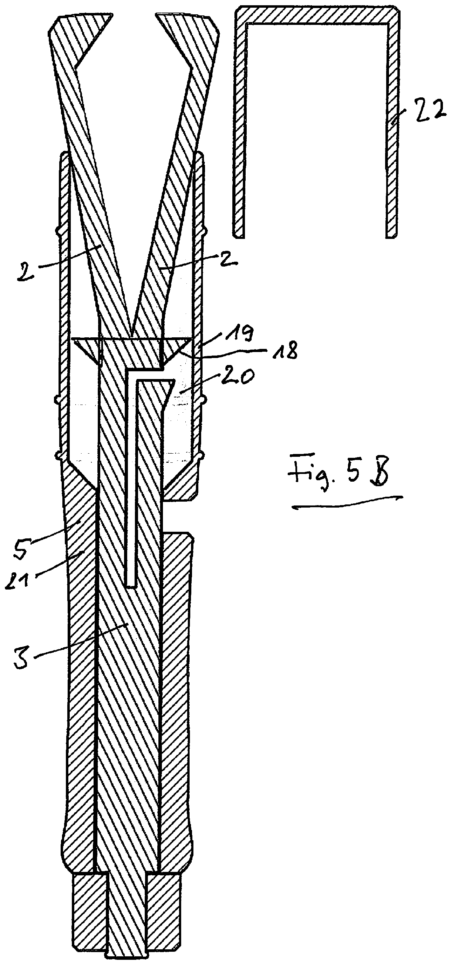

Figuren 5A und 5B zeigen bei einer abgewandelten Ausführungsform, dass an dem stabförmigen Griffabschnitt 3 im Bereich des Übergangs zu den Pinzettenhälften 2 ein ringförmiger Flansch 18 angeformt ist, dessen Außendurchmesser im wesentlichen mit dem Innendurchmesser des vorderen Wandabschnitts 19 übereinstimmt, um den Innenraum 20 zur Rückseite hin abzudichten. Der rückwärtige Abschnitt 21 der Hülse 5 hat einen querschnittlich rechteckigen Hohlraum, in dem der stabförmige Griffabschnitt 3 nicht-drehbar geführt ist.

Die Figuren 5A und 5B zeigen außerdem eine langgestreckte Kappe 22, die in dem in Figur 5B dargestellten Zustand auf den vorderen Endabschnitt der Hülse 5 aufgesetzt werden kann, wobei die Pinzettenhälften im entspannten Zustand aus der Hülse 5 herausragen.

## Patentansprüche

1. Zeckenzange zum Entfernen von Zecken aus der Haut,
mit einer äußeren Hülse (5), in der eine Pinzette (1) verschieblich gehalten ist, die zwei elastisch auseinander gespreizte Pinzettenhälften (2) aufweist, die einstückig mit einem stabförmigen Griffabschnitt (3) ausgebildet sind, dessen Endabschnitt (11) aus der Hülse (5) herausragt, wobei die Pinzettenhälften (2) aufeinander zulaufende Endabschnitte (4) zum Ergreifen einer Zecke haben und die Endabschnitte (4) in einem Ausgangszustand der Pinzette (1) mit Abstand voneinander vorne aus der Hülse (5) herausragen, wobei die Endabschnitte beim Andrücken der Hülse (5) an die Haut durch den vorderen Rand (14) der Hülse (5) zusammengezwängt werden, um die Zecke ergreifen zu können,
**dadurch gekennzeichnet,**
**dass** die in der Hülse (5) zurückgezogene Pinzette (1) durch eine Rasteinrichtung verrastet ist, die nur durch Einwärtsdrücken einer aus der Wand der Hülse (5) freigeschnittenen, federnden Zunge (15) freizugeben ist,
**dass** die Rasteinrichtung einen von dem Griffabschnitt (3) der Pinzette (1) freigeschnittenen federnden Steg (6) aufweist, der an seinem freien Ende eine Rastnase (7) hat, die im entspannten Zustand des Stegs (6) über die Außenkontur (10) des Griffabschnitts (3) hinaus nach außen ragt und radial nach innen einen Freiraum mindestens in der Breite der Rastnase (7) hat, und
**dass** die Hülse (5) in axialer Richtung vor ihrer freigeschnittenen Zunge (15) eine Aussparung (17) hat, in die die Rastnase (7) einrasten kann.

2. Zeckenzange nach Anspruch 1,
**dadurch gekennzeichnet,**
**dass** die Rastnase (7) eine etwa rechtwinklige abstehende vordere Stirnfläche (8) hat.

3. Zeckenzange nach Anspruch 1 oder 2
**dadurch gekennzeichnet,**
**dass** die freigeschnittene federnde Zunge (15) der Hülse bei in die Hülse (5) zurückgezogener Pinzette (1) deren freigeschnittenen Steg (6) im wesentlichen bis zu der Rastnase (7) überdeckt.

4. Zeckenzange nach einem der Ansprüche 1 bis 3,
**dadurch gekennzeichnet,**
**dass** der Griffabschnitt (3) querschnittlich eine Rechteckform hat und dass die Hülse (5) einen Abschnitt (21) mit entsprechendem rechteckigen Hohlraum aufweist, in dem der Griffabschnitt (3) nicht-drehbar geführt ist.

5. Zeckenzange nach einem der Ansprüche 1 bis 4,
**dadurch gekennzeichnet,**
**dass** auf den Endabschnitt (11) des Griffabschnitts (3) eine radial überstehende Kappe (12) aufgesetzt ist, die beim Vorschub der Pinzette (1) die vorgeschobene Endposition der Pinzette (1) begrenzt.

6. Zeckenzange nach einem der Ansprüche 1 bis 5,
**dadurch gekennzeichnet,**
**dass** sich die Pinzettenhälften (2) in der zurückgezogenen Position vollständig innerhalb der Hülse (5) befinden.

7. Zeckenzange nach einem der Ansprüche 1 bis 6,
**dadurch gekennzeichnet,**
**dass** die Hülse einen rückwärtigen Abschnitt (21) mit querschnittlich rechteckigem Hohlraum und einen anschließenden vorderen Abschnitt (19) mit querschnittlich kreisrundem Hohlraum aufweist.

8. Zeckenzange nach einem der Ansprüche 1 bis 7,
**dadurch gekennzeichnet,**
**dass** an dem Griffabschnitt (3) vor dem freigeschnittenen Steg (6) ein tellerförmiger Flansch (18) angeformt ist, dessen Rand einen minimalen Abstand von der Innenwand des vorderen Abschnitts (19) der Hülse (5) hat, so dass der Flansch (18) die Hülse (5) gegen den Durchgang einer Zecke abdichtet.

## Claims

1. Tweezers for removing ticks from the skin including an outer sleeve (5), in which pincers (1) are slidably mounted, which include two pincer halves (2) which are elastically spread apart from one another and which are integrally constructed with a rod-shaped gripping section (3), the end section (11) of which projects out of the sleeve (5), wherein the pincer halves (2) have end sections (4) extending towards one another for gripping a tick and the end sections (4) project forwardly out of the sleeve (5), in an initial position of the pincers (1), with a spacing from one another, wherein the end sections are forced together on pressing of the sleeve (5) against the skin through the front edge (14) of the sleeve (5) in order to be able to grip the tick, **characterised in that** when the pincers (1) are retracted into the sleeve they are locked by a locking device, which may be released only by pressing a resilient tongue (15) cut free from the wall of the sleeve (5) inwardly, that the locking device includes a resilient web (6) cut free from the gripping section (3) of the pincers (1), which has a locking lug (7) at its free end, which, in the relaxed state of the web (6), projects outwardly beyond the outer contour (10) of the gripping section (3) and, radially inwards, has a free space at least as broad as the locking lug (7) and that the sleeve (5) has a recess (17) before its cut free tongue (15) in the axial direction, into which the locking lug (7) can engage.

2. Tick tweezers as claimed in Claim 1, **characterised in that** the locking lug (7) has a front end surface (8) projecting approximately at right angles.

3. Tick tweezers as claimed in Claim 1 or 2, **characterised in that**, when the pincers (1) are withdrawn into the sleeve, the cut free resilient tongue (15) on the sleeve covers its cut free web (6) substantially up to the locking lug (7).

4. Tick tweezers as claimed in one of Claims 1 to 3, **characterised in that** the gripping section (3) has a rectangular shape in cross-section and that the sleeve (5) includes a section (21) with a correspondingly rectangular cavity, in which the gripping section (3) is non-rotatably guided.

5. Tick tweezers as claimed in one of Claims 1 to 4, **characterised in that** positioned on the end section (11) of the gripping section (5) there is a radially projecting cap (12), which limits the advanced end position of the pincers (1) when the pincers (1) are advanced.

6. Tick tweezers as claimed in one of Claims 1 to 5, **characterised in that** the pincer halves (2) are located wholly within the sleeve (5) in the retracted position.

7. Tick tweezers as claimed in one of Claims 1 to 6, **characterised in that** the sleeve has a rear section (21) with a cavity of rectangular cross-section and an adjacent front section (19) with a cavity of circular cross-section.

8. Tick tweezers as claimed in one of Claims 1 to 7, **characterised in that** integrally formed on the gripping section (3) in front of the cutaway web (6) there is a disc-shaped flange (18), the edge of which has a minimal spacing from the inner wall of the front section (19) of the sleeve (5) so that the flange (18) seals the sleeve (5) against the passage of a tick.

## Revendications

1. Pince à tiques destinée à retirer des tiques de la peau,
avec un manchon extérieur (5), dans lequel une pincette (1) est maintenue de manière à pouvoir coulisser, qui présente deux moitiés de pincette (2) écartées l'une de l'autre élastiquement, qui sont réalisées d'un seul tenant avec une section de préhension (3) en forme de barre, dont la section d'extrémité (11) dépasse du manchon (5), dans laquelle les moitiés de pincette (2) ont des sections d'extrémité (4) convergeant les unes vers les autres pour saisir une tique et les sections d'extrémité (4) dépassent, dans un état de départ de la pincette (1), du manchon (5) vers l'avant à distance l'une de l'autre, dans laquelle les sections d'extrémité sont rapprochées de manière forcée par le bord avant (14) du manchon (5) lorsque le manchon (5) est pressé contre la peau pour pouvoir saisir la tique,
**caractérisée en ce**
**que** la pincette (1) rentrée dans le manchon (5) est enclenchée par un dispositif d'enclenchement, qui ne doit être débloqué que par une pression vers l'intérieur d'une languette (15) à ressorts découpée de la paroi du manchon (5),
**que** le dispositif d'enclenchement présente une entretoise à ressorts (6) découpée de la section de préhension (3) de la pincette (1), qui a sur son extrémité libre un ergot d'enclenchement (7), qui dépasse vers l'extérieur du contour extérieur (10) de la section de préhension (3) dans l'état détendu de l'entretoise (6) et a vers l'intérieur radialement un espace libre au moins dans la largeur de l'ergot d'enclenchement (7), et
**que** le manchon (5) a, dans une direction axiale devant sa languette (15) découpée, un évidement (17), dans lequel l'ergot d'enclenchement (7) peut s'enclencher.

2. Pince à tiques selon la revendication 1,
**caractérisée en ce**
**que** l'ergot d'enclenchement (7) a une face frontale (8) avant dépassant à peu près à angle droit.

3. Pince à tiques selon la revendication 1 ou 2,
**caractérisée en ce**
**que** la languette à ressorts (15) découpée du manchon recouvre, lorsque la pincette (1) est rentrée dans le manchon (5), l'entretoise (6) découpée de celle-ci sensiblement jusqu'à l'ergot d'enclenchement (7).

4. Pince à tiques selon l'une quelconque des revendications 1 à 3,
**caractérisée en ce**
**que** la section de préhension (3) a dans la section transversale une forme rectangulaire, et que le manchon (5) présente une section (21) avec un espace creux rectangulaire correspondant, dans lequel la section de préhension (3) est guidée de manière non rotative.

5. Pince à tiques selon l'une quelconque des revendications 1 à 4,
**caractérisée en ce**
**qu'**est placé, sur la section d'extrémité (11) de la section de préhension (3), un capuchon (12) en porte-à-faux radialement, qui délimite la position finale avancée de la pincette (11) lors de l'avancement de la pincette (1).

6. Pince à tiques selon l'une quelconque des revendications 1 à 5,
**caractérisée en ce**
**que** les moitiés de pincette (2) se trouvent totalement à l'intérieur de la douille (5) dans la position rentrée.

7. Pince à tiques selon l'une quelconque des revendications 1 à 6,
**caractérisée en ce**
**que** la douille présente une section arrière (21) avec un espace creux rectangulaire dans la section transversale et une section avant (19) située dans le prolongement avec un espace creux rond circulaire dans la section transversale.

8. Pince à tiques selon l'une quelconque des revendications 1 à 7,
**caractérisée en ce**
**qu'**est formée sur la section de préhension (3) devant l'entretoise (6) découpée une bride (18) en forme de disque, dont le bord a une distance minimale de la paroi intérieure de la section avant (19) de la douille (5) de sorte que la bride (18) étanchéifie le manchon (5) pour empêcher le passage d'une tique.
